# EUROPÄISCHE PATENTANMELDUNG

(11) **EP 3 087 981 A1**
(43) Veröffentlichungstag der Anmeldung: **02.11.2016**
(21) Anmeldenummer: 15001297.9
(22) Anmeldetag: 30.04.2015
(51) Int. Cl.: A61K 31/122, A61P 31/10

(54) **HYGROPHORON B12 ZUR VERWENDUNG BEI DER BEHANDLUNG EINER MYKOSE**

(71) Anmelder: Leibniz-Institut für Pflanzenbiochemie (IPB), 06120 Halle (Saale) (DE)
(72) Erfinder: Arnold, Norbert, 06120 Halle (DE); Otto, Alexander, 06114 Halle (DE); Bette, Eileen, 06110 Halle (DE); Westermann, Bernhard, 06120 Halle (DE); Lübken, Tilo, 04347 Leipzig (DE); Wessjohann, Ludger, 06120 Halle (DE)
(74) Vertreter: Müller-Boré & Partner Patentanwälte PartG mbB

(57) **Zusammenfassung**

Die vorliegende Erfindung betrifft Hygrophoron B¹² oder dessen Enantiomer, Epimere oder Diastereomere zur Verwendung bei der Behandlung einer Mykose.

## Beschreibung

Die vorliegende Erfindung betrifft Hygrophoron B¹² oder dessen Enantiomer, Epimere oder Diastereomere zur Verwendung bei der Behandlung einer Mykose.

Als Mykose bezeichnet man eine parasitäre Infektionskrankheit des lebenden Gewebes durch Pilze. Erreger können Dermatophyten (Fadenpilze), Hefen (Sprosspilze) und Schimmelpilze sein. Ein im Handel erhältliches, bekanntes Mittel zur Behandlung von Mykosen ist Terbinafin, welches ein Allylamin-Derivat ist.

Es besteht jedoch weiterer Bedarf an Arzneimitteln zur Behandlung von Mykose.

Diese Aufgabe wird durch die in den Ansprüchen gekennzeichneten Ausführungsformen gelöst.

Gemäß der vorliegenden Erfindung wird Hygrophoron B¹² der Formel I 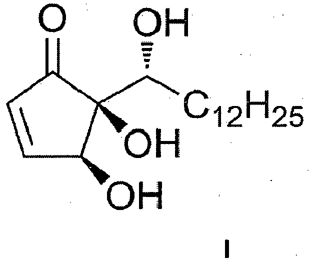 oder dessen Enantiomer, Epimere oder Diastereomere zur Verwendung bei der Behandlung einer Mykose bereitgestellt.

Pilze der Gattung Schnecklinge (*Hygrophorus*) leben in Symbiose mit verschiedenen Laub- und Nadelbäumen. Viele dieser Arten sind dadurch gekennzeichnet, dass ihre Fruchtkörper von einer kräftigen Schleimschicht überzogen sind. Bei eingehenden Untersuchungen der Fruchtkörper der Schnecklinge ist aufgefallen, dass diese kaum von parasitischen Pilzen befallen werden. Diese ökologische Beobachtung führte zu Untersuchungen von Schnecklingen im Hinblick auf die Entwicklung von bioziden Aktivitäten. In DE 10 2004 009 185 A1 wurden aus Rohextrakten der Schnecklinge neue Verbindungen, sogenannte Hygrophorone, isoliert und charakterisiert. Diese Hygrophorone weisen einen Cyclopentenon-Ring als Grundgerüst auf. In DE 10 2004 009 185 A1 wurden einige Hygrophorone bezüglich ihrer Bioaktivität gegenüber *Cladosporium cucumerinum. Staphylococcus aureus, Enterococcus faecalis* und *Streptococcus pneumoniae* untersucht. Dabei zeigten die getesteten Hygrophorone starke antibiotische und antimykotische Eigenschaften. In DE 10 2004 009 185 A1 wurde jedoch nicht Hygrophoron B¹² beschrieben.

Bette et al., European Journal of Organic Chemistry, 2015, Vol. 2015, Issue 11, Seite 2357 bis 2365, offenbart neben vielen anderen Verbindungen Hygrophoron B¹², das als Alkylseitenkette -C₁₂H₂₅ aufweist. In Bette et al. wurde die Gewinnung von Hygrophoron B¹² zum einen durch Extraktion aus dem Pilz *Hygrophorus abieticola* und zum anderen durch Totalsynthese aufgezeigt. Hygrophoron B¹² wurde in Bette et al. aber nicht bezüglich irgendeiner Bioaktivität untersucht.

Im erfindungsgemäß verwendeten Hygrophoron B¹² weisen die zwei endocyclischen Hydroxy-Gruppen eine *cis*-Konfiguration auf, während die exocyclische Hydroxy-Gruppe am Kohlenstoffatom C-6 eine *anti*-Konfiguration relativ zur Hydroxy-Gruppe am Kohlenstoffatom C-5 zeigt. Dementsprechend wurde in Bette et al. Hygrophoron B¹² als (+)-*cis*-4,5-Dihydroxy-5-(1-hydroxytridecyl)cyclopent-2-enon bezeichnet. Die vorliegende Erfindung ist jedoch nicht auf die Verwendung von Hygrophoron B¹² zur Behandlung einer Mykose beschränkt. Zusätzlich können auch das Enantiomer zum natürlichen Hygrophoron B¹² sowie Epimere und Diastereomere des Naturstoffs verwendet werden. Das Enantiomer und Beispiele von Stereoisomeren wurden in Bette et al. synthetisiert. Neben den trihydroxyfunktionalisierten carbocyclischen Produkten können auch die di- und mono-hydroxylierten Derivate in ihren Epimer- und Enantiomerenformen Verwendung finden.

Unter dem hierin verwendeten Begriff "Mykose" wird eine parasitäre Infektionskrankheit des lebenden Gewebes durch Pilze verstanden.

Der Lokalisation folgend lassen sich Mykosen in oberflächliche Mykosen, wie beispielsweise der Haut, der Nägel und der Schleimhäute, sowie systemische Mykosen, die die inneren Organe betreffen, einteilen.

Gemäß einer bevorzugten Ausführungsform der vorliegenden Erfindung wird Hygrophoron B¹² oder dessen Enantiomer, Epimere oder Diastereomere zur Verwendung bei der Behandlung einer Mykose der Haut eingesetzt.

Im Rahmen der vorliegenden Erfindung kann Hygrophoron B¹² oder dessen Enantiomer, Epimere oder Diastereomere zur Behandlung von Menschen oder Tieren eingesetzt werden. Dabei sind die Tiere, die mit dem erfindungsgemäß verwendeten Hygrophoron B¹² oder dessen Enantiomer, Epimere oder Diastereomere behandelt werden können, nicht besonders eingeschränkt. Beispielsweise können Haustiere, insbesondere Hunde oder Katzen, oder Nutzvieh jedweder Art mit Hygrophoron B¹² oder dessen Enantiomer, Epimere oder Diastereomere behandelt werden. Gemäß einer bevorzugten Ausführungsform der vorliegenden Erfindung wird Hygrophoron B¹² oder dessen Enantiomer, Epimere oder Diastereomere zur Behandlung einer Mykose bei Hunden eingesetzt.

Wie vorstehend bereits ausgeführt, sind Erreger einer Mykose Dermatophyten (Fadenpilze), Hefen (Sprosspilze) und Schimmelpilze. Gemäß einer bevorzugten Ausführungsform der Erfindung wird Hygrophoron B¹² oder dessen Enantiomer, Epimere oder Diastereomere zur Verwendung bei der Behandlung einer Mykose eingesetzt, die von Dermatophyten oder Hefen verursacht wird.

Dermatophyten liegen in einer der Gattungen *Epidermophyton, Microsporum, Candida, Trichophyton, Rhodotorula, Scopulariopsis oder Aspergillus* vor.

Die Gattung *Epidermophyton, wie z.B. die Art Epidermophyton floccosum,* hat als Hautpilz eine medizinisch relevante Bedeutung, da er ein Erreger von Hautpilzerkrankungen ist. Er befällt ausschließlich den Menschen; betroffen ist vorrangig die Haut, seltener auch die Nägel.

Arten der Gattung *Microsporum,* wie z.B. *Microsporum canis,* sind ein auf der Haut vorkommender Pilz. Er ist ein vor allem bei Katzen und Hunden häufiger Erreger einer Hautpilzerkrankung, kann aber auch beim Menschen und bei anderen Säugetieren eine Erkrankung auslösen.

Arten der Gattung *Trichophyton,* wie z.B. *Trichophyton tonsurans,* können Pilzerkrankungen der Haut sowie von Kopf- und Barthaaren auslösen.

Arten der Gattung *Candida* und *Rhodotorula,* wie z.B. *Candida albicans* und *Rhodotorula rubra,* haben die Fähigkeit, sowohl in Hefe- als auch in Hyphenform zu wachsen, was ihre Invasion von vorgeschädigter Haut ermöglicht.

Schimmelpilze aus den Gattungen *Scopulariopsis* oder *Aspergillus,* wie z.B. *Scopulariopsis brevicaulis* oder *Aspergillus* spp., können eine Nagelpilzerkrankung verursachen. Diese Onychomykose weist keine Selbstheilungstendenz auf und kann zum Ausgangspunkt weiterer Mykoseherde der Haut werden.

Gemäß der vorliegenden Erfindung unterliegen die Hefen, die eine Mykose verursachen können, keiner besonderen Einschränkung. Gemäß einer bevorzugten Ausführungsform der vorliegenden Erfindung wird Hygrophoron B¹² oder dessen Enantiomer, Epimere oder Diastereomere bei der Behandlung einer Mykose verwendet, die von einer der Hefen *Malassezia,* insbesondere *Malassezia furiur, Malassezia pachydermatis, Malassezia sympodialis, Malassezia globosa, Malassezia obtusa, Malassezia restricta, Malassezia slooffiae, Malassezia dermatis, Malassezia japonica, Candida albicans* oder *Rhodotorula rubra* verursacht wird.

*Malassezia* kommen auf der Haut bei den meisten Menschen und Tieren vor und können lebensbedrohliche Fungämien, d.h. das Vorkommen von Pilzen oder Pilzelementen im Blut nach Einbrechen eines pilzbedingten Prozesses in die Blutbahn, oder andere nosokomiale Infektionen (Krankenhausinfektionen) in immungeschwächten Patienten, insbesondere bei Neugeborenen, auslösen. Während diese Krankheit im Menschen meist durch *Malassezia furfur* verursacht wird, kann die Erkrankung auch eine Folge von *Malassezia pachydermatis* sein, für die Hunde natürliche Wirte sind. In einigen Fällen wurde die Ursache der Infektion eines Menschen auf Hunde zurückgeführt, die von Pflegekräften gehalten wurden. Verstärkte Hefenbildung kann entstehen, wenn *Staphylococcus* spp. anwesend sind. *Malassezia pachydermatis* ist die wichtigste Malassezia-Art beim Hund und löst die Malassezia-Dermatitis aus. Die lipophile Hefe findet sich vor allem in fettiger Haut und Gehörgängen mit viel Ohrenschmalz.

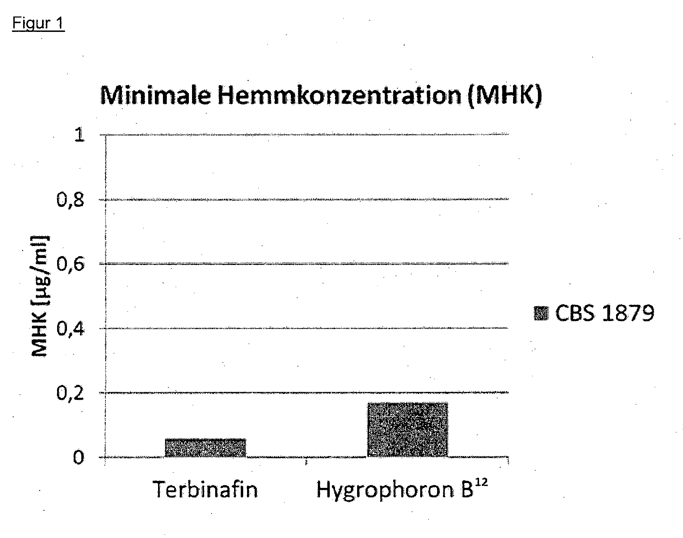

Einer besonders bevorzugten Ausführungsform der Erfindung entsprechend wird Hygrophoron B¹² oder dessen Enantiomer, Epimere oder Diastereomere zur Verwendung bei der Behandlung einer Mykose eingesetzt, die durch *Malassezia pachydermatis* ausgelöst wird. Die Evaluierung der biologischen Eigenschaften des erfindungsgemäß verwendeten Hygrophorons B¹² ergab, dass Hygrophoron B¹² stark antimykotische Eigenschaften aufweist. Insbesondere weist Hygrophoron B¹² starke Aktivität gegen *Malassezia pachydermatis* auf. Figur 1 zeigt eine überraschend starke *in vitro* Aktivität des erfindungsgemäß verwendeten Hygrophorons B¹² gegen *Malassezia pachydermatis,* wobei die Aktivität mit dem Arzneistoff Terbinafin vergleichbar ist. Terbinafin ist ein synthetisches Antimykotikum und seit vielen Jahren das meist verordnete Präparat. In Figur 1 bezeichnet MHK die minimale HemmKonzentration (MHK). Sie ist die niedrigste Konzentration einer Substanz, bei der die Vermehrung von Mikroorganismen mit bloßem Auge nicht wahrgenommen werden kann. Die MHK wird mit einem sogenannten Titerverfahren bestimmt. Dabei wird die Konzentration von einem Wirkstoff (hier Terbinafin und Hygrophoron B¹²) bestimmt, der das Wachstum von *Malassezia pachydermatis* gerade noch hemmt. Die MHK wird in Mikrogramm pro Milliliter (µg/ml) angegeben.

Die Behandlung von Mykosen mit Hygrophoron B¹² ist vorteilhaft, da es lipophil ist und im Hautfett und Ohrenschmalz löslich ist. Da Hygrophoron B¹² im Vergleich zu etablierten Präparaten, wie beispielsweise Terbinafin, einen neuen Wirkmechanismus vermuten lässt, sind bisherige Resistenzen umgehbar.

Eine weitere bevorzugte Ausführungsform der Erfindung ist Hygrophoron B¹² oder dessen Enantiomer, Epimere oder Diastereomere zur Verwendung bei der Behandlung einer Mykose, die durch *Malassezia furfur* verursacht wird. Da die fakultativ humanpathogene Hefe *Malassezia furfur* eng mit *Malassezia pachydermatis* verwandt ist, wirkt das erfindungsgemäß verwendete Hygrophoron B¹² oder dessen Enantiomer, Epimere oder Diastereomere auch gegen *Malassezia furfur* antimykotisch. Somit kann auch die Erkrankung *Pityriasis versicolor,* die von *Malassezia furfur* hervorrufen wird, mit dem erfindungsgemäß verwendeten Hygrophoron B¹² oder dessen Enantiomer, Epimere oder Diastereomere behandelt werden.

Einer weiteren Ausführungsform der vorliegenden Erfindung entsprechend kann Hygrophoron B¹² oder dessen Enantiomer, Epimere oder Diastereomere zur Behandlung einer Mykose verwendet werden, die von der Hefe *Candida albicans* verursacht wird. *Candida albicans* kann die Soor-Krankheit auf Haut sowie Schleimhäuten auslösen. Candida-Arten, an erster Stelle *Candida parapsilosis,* gefolgt von *Candida guilliermondii* und *Candida albicans,* können auch Hautanhangsgebilde, besonders der Nägel (Onychomykose), aber auch des Nagelbetts sowie der perionychialen Region (chronische Paronychie) befallen. Die Onychomykose ist manchmal eine echte Monoinfektion, oft jedoch eine Mischinfektion von einem Dermatophyten mit einem oder mehreren Sprosspilzen.

Die im Rahmen der vorliegenden Erfindung offenbarten Verwendungen betreffen auch die Formulierung des erfindungsgemäß verwendeten Hygrophorons B¹² oder dessen Enantiomer, Epimere oder Diastereomere in geeigneten Formen, z.B. in der Form von pharmazeutischen Zubereitungen, umfassend Hygrophoron B¹² oder dessen Enantiomer, Epimere oder Diastereomere sowie einen pharmazeutisch akzeptablen Trägerstoff. In Übereinstimmung mit der vorliegenden Erfindung können daher Lösungen, Suspensionen, Emulsionen, Salben, Tabletten, Kapseln, Lösungen oder Emulsionen zum Sprüheinsatz usw. bereitgestellt werden, so dass das erfindungsgemäß verwendete Hygrophoron B¹² oder dessen Enantiomer, Epimere oder Diastereomere in geeigneter Weise in den jeweiligen Einsatzbereichen eingesetzt werden kann. Bevorzugt ist die Verwendung eines fetthaltigen Trägerstoffs in der pharmazeutischen Zubereitung.

Im Rahmen der vorliegenden Erfindung kann das Hygrophoron B¹² oder dessen Enantiomer, Epimere oder Diastereomere auch mit weiteren Hilfsstoffen und anderen pharmazeutisch aktiven Verbindungen kombiniert werden, auch besonders im Gemisch mit anderen Antimykotika oder Antibiotika (antibakterielle Substanzen). Bevorzugt sind dabei topische Zubereitungen, besonders fettbasierte Salben.

Das erfindungsgemäß verwendete Hygrophoron B¹² kann mit Hilfe der im Stand der Technik verwendeten Verfahren erhalten werden, insbesondere durch Extraktion aus Fruchtkörpern von Pilzen der Gattung Schnecklinge (*Hygrophorus*)*,* durch biotechnologische Verfahren oder durch Synthese. Bette et al. beschreibt zum einen die Extraktion von Hygrophoron B¹² aus gefrorenen Fruchtkörpern des Pilzes *Hygrophorus abieticola* mit Ethylacetat. Zum anderen offenbart Bette et al. die Totalsynthese von Hygrophoron B¹² in 9 Stufen, die unter anderem eine asymmetrische Sharpless-Dihydroxylierung und zwei diastereoselektive Aldol-Reaktionen umfasst. Im Zuge dieser Synthese wurden auch das Enantiomer sowie Beispiele von Stereoisomeren des natürlichen Hygrophorons B¹² dargestellt. Stereoisomere des Hygrophorons B¹² können auch durch biotechnologische Verfahren hergestellt werden.

Die Behandlung von Mykosen mit dem erfindungsgemäß verwendeten Hygrophoron B¹² oder dessen Enantiomer, Epimere oder Diastereomere ist vorteilhaft, da Hygrophoron B¹² oder dessen Enantiomer, Epimere oder Diastereomere im Vergleich zu etablierten Präparaten einen neuen Wirkmechanismus vermuten lässt und somit verminderte Resistenzphänomene zu erwarten sind. Insbesondere zeigt Hygrophoron B¹² eine hohe antimykotische Aktivität gegen die Hefe *Malassezia pachydermatis,* welche die wichtigste *Malassezia-Art* beim Hund ist und die Malassezia-Dermatitis auslöst. Somit besitzt das erfindungsgemäß verwendete Hygrophoron B¹² in der Behandlung von Mykosen ein großes ökonomisches Potential, da die Tiermedizin einer der Wachstumsbereiche im Arzneimittelgeschäft ist, und global mit Tierarzneien und -impfstoffen deutlich mehr als 15 Milliarden Euro im Jahr umgesetzt werden. Neben einer Anwendung im veterinärmedizinischen Bereich ist aber auch eine Anwendung im Bereich der Humanmedizin möglich, da *Malassezia pachydermatis* mit der fakultativ humanpathogenen Hefe *Malassezia furfur* eng verwandt ist.

Figur 1 zeigt Ergebnisse bezüglich der minimalen Hemm-Konzentration (MHK) von dem handelsüblichen Terbinafin im Vergleich zum erfindungsgemäß verwendeten Hygrophoron B¹² gegenüber dem Erreger *Malassezia pachydermatis.*

Das nachstehende Beispiel dient als weitere Erläuterung der vorliegenden Erfindung, ohne darauf beschränkt zu sein.

### Beispiel

Die Aktivität von Hygrophoron B¹² und Terbinafin gegen *Malassezia pachydermatis* wurde in einem Verdünnungsassay in 96-Well Mikrotiterplatten ermittelt. Terbinafin bzw. Hygrophoron B¹² wurden in DMSO gelöst, dann mit RPMI 1640 Medium (Sigma Aldrich Art. R8755, enthaltend zusätzlich 0.1% Tween 40 und 0.4% Glycerol) auf folgende Endkonzentrationen verdünnt: 42 µg/ml, 14 µg/ml, 4,66 µg/ml, 1,55 µg/ml, 0,52 µg/ml, 0,17 µg/ml, 0,058 µg/ml und 0,019 µg/ml.
Der Malassezia-Stamm CBS 1879 (Kultur bezogen von Centraalbureau voor Schimmelcultures (CBS) Fungal Biodiversity Centre - an institute of the Royal Netherlands Academy of Arts and Sciences (KNAW), Utrecht /The Netherlands; www.cbs.knaw.nl/collections/BioloMICS.aspx?Table=CBS strain database&Name=CBS+1879&Fields=All&ExactMatch=T) wurde auf Sabouraud Dextrose Agarplatten (SDA) (Sabouraud Dextrose Bouillon Merck Art.1.08339.0500, 30g/L; Agar-Agar Roth Art. 5210.3, 15 g/L) kultiviert und anschließend mit einem sterilen Wattestäbchen von der Oberfläche entfernt und in RPMI 1640 Medium gegeben. Die erhaltene Suspension wurde auf eine Konzentration von ca. 1,0x10³ koloniebildenden Einheiten (KBE) pro 1 ml eingestellt. 50 µl der Substanzlösungen und 50 µl der Malassezia-Suspension wurden im jeweiligen Well gemischt und für 2 bis 4 Tage bei 37 °C inkubiert. Die Evaluierung der Hemmung erfolgte per *occulum.*

Die Konzentration, bei der noch eine Inhibierung des Wachstums von *Malassezia pachydermatis* festzustellen war, wird als minimale Hemmkonzentration (MHK) bezeichnet. Es zeigte sich eine MHK von Hygrophoron B¹² von 0,17 µg/mL gegen das *Malassezia pachydermatis* Isolat CBS 1879 (Referenz Terbinafin 0,058 µg/mL).

## Patentansprüche

1. Hygrophoron B¹² der Formel I 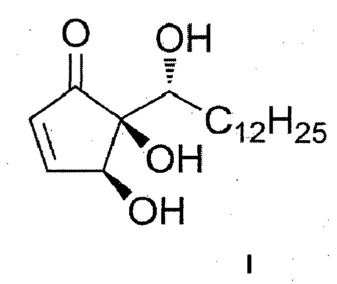 oder dessen Enantiomer, Epimere oder Diastereomere zur Verwendung bei der Behandlung einer Mykose.

2. Hygrophoron B¹² oder dessen Enantiomer, Epimere oder Diastereomere zur Verwendung gemäß Anspruch 1, wobei eine Mykose der Haut vorliegt.

3. Hygrophoron B¹² oder dessen Enantiomer, Epimere oder Diastereomere zur Verwendung gemäß Anspruch 1 oder 2, wobei die Mykose Mensch oder Tier betrifft.

4. Hygrophoron B¹² oder dessen Enantiomer, Epimere oder Diastereomere zur Verwendung gemäß einem der Ansprüche 1 bis 3, wobei die Mykose von Dermatophyten oder Hefen verursacht wird.

5. Hygrophoron B¹² oder dessen Enantiomer, Epimere oder Diastereomere zur Verwendung gemäß Anspruch 4, wobei die Hefe eine von *Malassezia furfur, Malassezia pachydermatis, Malassezia sympodialis, Malassezia globosa, Malassezia obtusa, Malassezia restricta, Malassezia slooffiae, Malassezia dermatis, Malassezia japonica, Candida albicans* oder *Rhodotorula rubra ist.*

6. Hygrophoron B¹² oder dessen Enantiomer, Epimere oder Diastereomere zur Verwendung gemäß Anspruch 4, wobei die Dermatophyten eines aus den Gattungen *Epidermophyton, Microsporum, Candida, Trichophyton, Rodotorula, Scopulariopsis* oder *Aspergillus* sind.

7. Hygrophoron B¹² oder dessen Enantiomer, Epimere oder Diastereomere zur Verwendung gemäß einem der Ansprüche 1 bis 6, wobei das Hygrophoron B¹² oder dessen Enantiomer, Epimere oder Diastereomere in einer Zubereitung formuliert ist, weiter umfassend einen pharmazeutisch akzeptablen Trägerstoff.

8. Hygrophoron B¹² oder dessen Enantiomer, Epimere oder Diastereomere zur Verwendung gemäß Anspruch 7, wobei der Trägerstoff ein fetthaltiger Trägerstoff ist.

9. Hygrophoron B¹² oder dessen Enantiomer, Epimere oder Diastereomere zur Verwendung gemäß Anspruch 7 oder 8, wobei die Zubereitung topisch angewendet wird.
